# FASCICULE DE BREVET EUROPEEN

(11) **EP 0 797 977 B1**
(45) Date de publication et mention de la délivrance du brevet: **07.01.1999**
(21) Numéro de dépôt: 97400259.4
(22) Date de dépôt: 05.02.1997
(51) Int. Cl.: A61K 7/043

(54) **Composition cosmétique à appliquer sur un support kératinique comprenant au moins deux polymères et utilisation**
Kosmetisches Präparat zum Aufbringen auf Keratinmaterial das zwei Polymere enthält und Anwendung
Cosmetic composition for applying on keratinic material comprising at least two polymers and use

(30) Priorité: 13.03.1996 FR 9603176
(43) Date de publication de la demande: 01.10.1997
(73) Titulaire: L'OREAL, 75008 Paris (FR)
(72) Inventeur: Ramin, Roland, 91760 Itteville (FR); Mellul, Myriam, 94240 l'Hay les Roses (FR)
(74) Mandataire: Dodin, Catherine

(56) Documents cités:
- EP-A- 0 230 364
- EP-A- 0 391 322
- EP-A- 0 627 212
- EP-A- 0 661 311

## Description

La présente invention concerne une composition, notamment cosmétique, à appliquer sur un support kératinique, comprenant au moins deux polymères filmogènes.

De façon générale, un vernis à ongles, pour présenter de bonnes propriétés cosmétiques, doit avoir une bonne adhérence sur la surface de l'ongle; le film de vernis doit être homogène et brillant, et présenter une certaine flexibilité tout en étant résistant, en vue d'éviter les craquelures et l'écaillement.
Pour obtenir de telles propriétés, il est nécessaire que le film de vernis soit suffisamment mou de façon à pouvoir supporter les déformations de la matrice de l'ongle et les chocs. Mais le film doit être également suffisamment dur pour éviter les rayures et les craquelures et doit également conserver une certaine brillance.

Cette double exigence n'est généralement pas remplie avec les vernis usuels, qui comprennent un seul polymère filmogène.
Une solution consiste à utiliser plusieurs polymères filmogènes en mélange dans la composition de vernis à ongles.
Ainsi, la demande de brevet EP-A-391 322 décrit un vernis à ongles comprenant une dispersion de polyuréthanne et de résine acrylique.
Toutefois, lors de l'application du vernis sur l'ongle et après filmification, les deux polymères forment dans le film obtenu un mélange homogène (alliage). Ce film présente des propriétés cosmétiques intermédiaires entre celles des polymères du mélange. Un tel vernis ne permet donc pas d'obtenir les meilleures propriétés requises, ni de bénéficier des propriétés propres à chacun des polymères.

Le but de la présente invention est de proposer une composition, notamment cosmétique, susceptible d'être appliquée sur un support kératinique, apte à fournir un film à la fois suffisamment mou et suffisamment dur, et qui présente ainsi de bonnes propriétés cosmétiques.

Ainsi, la présente invention a pour objet une composition susceptible d'être appliquée sur un support kératinique, caractérisée par le fait qu'elle comprend un premier polymère filmogène ayant une tension superficielle γ₁ proche de la tension superficielle du support sur lequel ladite composition est appliquée, et un second polymère filmogène ayant une tension superficielle γ₂ inférieure à γ₁, les deux polymères étant incompatibles entre eux.

Un autre objet de l'invention est relatif à l'utilisation dans une composition à appliquer sur un support kératinique d'un premier polymère filmogène ayant une tension superficielle γ₁ proche de la tension superficielle dudit support kératinique sur lequel ladite composition est appliquée, et d'un second polymère filmogène ayant une tension superficielle γ₂ inférieure à γ₁, les deux polymères filmogènes étant incompatibles entre eux, en particulier afin d'obtenir sur ledit support un film stratifié.

Le Demandeur a constaté qu'après application de la composition sur le support kératinique et séchage, on obtient un film constitué de deux polymères stratifiés, le premier polymère constituant la couche au contact du support kératinique et le deuxième polymère constituant la couche au contact de l'air.
Toutefois, la séparation entre les deux couches de polymères est plus ou moins franche.
Ainsi, par premier polymère constituant la couche au contact du support kératinique, on entend qu'une majorité du premier polymère, par rapport au deuxième polymère, est en contact avec la surface dudit support.
De façon parallèle, le deuxième polymère est en majorité présent dans la deuxième couche au contact de l'air.
Cette répartition des deux polymères dans les deux couches n'exclut pas, par exemple, la présence d'inclusion du deuxième polymère dans la couche au contact dudit support dans laquelle le premier polymère est présent en majorité, ou inversement.
La composition selon l'invention constitue donc une composition auto-stratifiante.

Dans le film stratifié, on constate que la couche au contact du support kératinique présente une bonne adhérence avec la surface dudit support et une bonne souplesse; la couche supérieure au contact de l'air présente une bonne brillance, une bonne dureté, une bonne tenue à l'usure et une bonne résistance.

La composition selon l'invention comprend donc au moins deux polymères filmogènes incompatibles entre eux, ayant chacun des tensions superficielles particulières.

Par tension superficielle d'un polymère, on entend la tension superficielle d'un film sec constitué uniquement dudit polymère.
La mesure de tension superficielle peut notamment être déterminée selon la méthode des angles de contact. Cette méthode consiste à déposer une goutte d'eau et une goutte de diiodométhane sur le film de polymère, à mesurer l'angle formé par chaque goutte avec le film, puis à calculer la tension superficielle dudit film d'après la méthode décrite dans "Double liaison - Chim. Peint. 82." Vol.29, pages 263 à 268.

Par "tension superficielle γ₁ proche de la tension superficielle du support kératinique", on entend une tension superficielle γ₁ telle que : 0,9 x γₛᵤₚₚₒᵣₜ ≤ γ₁ ≤ 1,1 x γₛᵤₚₚₒᵣₜ , γₛᵤₚₚₒᵣₜ désignant la tension superficielle dudit support kératinique.

Le premier polymère ayant une tension superficielle γ₁, tout comme le second polymère filmogène ayant une tension superficielle γ₂, peut être soit un seul polymère, soit un mélange homogène (c'est-à-dire ne formant qu'une seule phase dans le film sec obtenu avec ledit mélange) de deux ou plusieurs polymères, le mélange ayant alors respectivement une tension superficielle γ₁ ou γ₂.

Par "polymères incompatibles entre eux", on entend des polymères qui forment au moins deux phases différentes dans le film sec obtenu après l'application de la composition selon l'invention. Les polymères peuvent être totalement incompatibles ou partiellement incompatibles, c'est-à-dire que seulement une partie de la ou des chaînes du premier polymère sont incompatibles avec une partie de la ou des chaînes du second polymère. Lorsque les polymères sont totalement incompatibles, la séparation des deux phases dans le film sec est franche. Lorsque les polymères sont partiellement incompatibles, la séparation des deux phases dans le film sec n'est pas franche.

Le support kératinique peut être l'ongle, le cil, le sourcil ou le cheveu.

De préférence, lorsque le support kératinique est l'ongle, on peut choisir γ₁ allant de 30 à 35 mJ/m², et γ₂ , inférieur à γ₁ , allant de 22 à 32 mJ/m².

Lorsque le support kératinique est le cheveu, le cil ou le sourcil, γ₁ va de préférence de 28 à 33 mJ/m² et γ₂ est inférieur à γ₁ et va de 22 à 32 mJ/m².

Au moins deux des polymères présents dans la composition selon l'invention doivent être incompatibles entre eux, c'est-à-dire qu'ils doivent former deux phases différentes dans le film sec obtenu après l'application de la composition sur le support.

La composition selon l'invention peut comprendre notamment un troisième polymère, voire plusieurs autres polymères, dont la tension superficielle γ₃ est inférieure à γ₂, ce ou ces polymères additionnels étant aptes à former d'autres couches de polymères superposées à celles formées par les premier et second polymères selon l'invention.

Les polymères filmogènes selon l'invention peuvent être choisis parmi les polymères radicalaires, les polycondensats, les polymères d'origine naturelle et leurs mélanges.

Les polymères radicalaires peuvent être choisis parmi les polymères acryliques et/ou les polymères vinyliques.
Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.

Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.
On utilise de préférence des polymères acryliques obtenus par copolymérisation de monomères à insaturation éthylénique contenant des groupements hydrophiles, de préférence de nature non ionique, tels que l'acrylate d'hydroxyéthyle, l'acrylate de 2-hydroxy propyle, le méthacrylate d'hydroxyéthyle, le méthacrylate de 2-hydroxy propyle.
Les polymères vinyliques peuvent résulter de l'homopolymérisation ou de la copolymérisation de monomères choisi parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle.
Les polymères radicalaires selon l'invention peuvent également être substitués par de atomes d'halogènes, et notamment par des atomes de fluor.

Selon l'invention, les polycondensats peuvent être choisis parmi les polyuréthannes, les polyesters, les polyesters amides, les polyamides, les polyesters à chaîne grasse, les résines époxyesters et leurs mélanges.
Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol. Comme diol aromatique, on peut utiliser le 4,4'-(1-méthylpropylidène)bisphénol. Comme polyols, on peut utiliser le glycérol, le pentaérythritol, le sorbitol, le triméthylol propane.
Les polyesters amides peuvent être obtenus de manière analogue aux polyesters, par polycondensation de diacides avec des diamines ou des amino alcools. Comme diamine, on peut utiliser l'éthylène diamine, l'hexaméthylène-diamine, la méta- ou para-phénylène diamine. Comme aminoalcool, on peut utiliser la monoéthanolamine.
Comme monomère porteur de groupement anionique pouvant être utilisé lors de la polycondensation, on peut citer par exemple l'acide diméthylol propionique, l'acide trimellitique ou un dérivé tel que l'anhydride trimellitique, le sel de sodium de l'acide pentanediol 3-sulfonique, le sel de sodium de l'acide 5-sulfo 1,3-benzènedicarboxylique.
Les polyesters à chaîne grasse peuvent être obtenus par l'utilisation de diols à chaîne grasse lors de la polycondensation.
Les résines époxyesters peuvent être obtenues par polycondensation d'acides gras avec un condensat aux extrémités α, ω - diépoxy. De tels polymères sont notamment décrits dans K. KRISHNAMURTI, Progress in organic coatings, 11 (1983), 167-197.
Les polycondensats selon l'invention peuvent également être substitués par de atomes d'halogènes, et notamment par des atomes de fluor.

Comme polymère d'origine naturelle, on peut citer la shellac et la gomme de sandaraque.

Selon l'invention, la teneur en matière sèche de polymères filmogènes peut aller de 5 % à 50 %, et de préférence de 25 % à 35 %, en poids par rapport au poids total de la composition.

Le mélange de polymères filmogènes présent dans la composition peut être constitué, en matière sèche, de 5 % à 95 %, et de préférence de 30 % à 80 %, d'un premier polymère filmogène, et de 5 % à 95 %, et de préférence de 20 % à 70 %, d'un second polymère filmogène. De manière préférentielle, le mélange de polymères filmogènes peut être constitué de 50 % du premier polymère filmogène et de 50 % du second polymère filmogène.

La composition selon l'invention peut également comprendre, en plus des polymères filmogènes, des plastifiants qui permettent de régler la flexibilité du film sans affaiblir sa résistance physique.
Les plastifiants utilisables sont ceux couramment employés dans les compositions de vernis à ongles. Comme plastifiants, on peut citer les phtalates de dibutyle, de dioctyle, de di-isobutyle, de diméthoxyéthyl, les benzoates de benzyle, de glycéryle; les citrates de triéthyle, de tributyle, l'acétyl-citrate de tributyle; les phosphates de tributyle, de triphényle; les glycols; le camphre ainsi que leurs dérivés et leurs mélanges. Les plastifiants peuvent être généralement présents à une teneur allant de 1 % à 30 % en poids par rapport au poids total de la composition.

Conformément à l'invention, les polymères filmogènes peuvent être dissous ou dispersés dans le milieu de la composition. Ledit milieu cosmétiquement acceptable peut être constitué de solvant ou de mélange de solvant qui sont habituellement utilisés dans les compositions de vernis à ongles. Comme solvants organiques, on peut citer les cétones, comme l'acétone, la méthyléthylcétone, la méthyl-isobutyl-cétone ; les éthers de glycol ; les alcools comme l'éthanol, le n-butanol, le n-propanol, l'isopropanol ; les acétates comme l'acétate de butyle, d'éthyle, d'isopropyle, l'acétate de méthoxy-2-éthyle ; les hydrocarbures linéaires ou ramifiés tels que l'hexane ou l'octane ; ou encore les hydrocarbures aromatiques tels que le xylène et le toluène. On peut également utiliser de l'eau, seule ou en mélange avec un ou plusieurs solvants.
De préférence, on utilise les acétates de butyle et d'éthyle.

La composition selon l'invention peut contenir des adjuvants couramment utilisés en cosmétique. On peut citer à titre d'exemple d'adjuvants les colorants, les pigments, les nacres, les laques, les agents anti-UV, les agents épaississants, les parfums, les agents anti-mousse, les agents tensioactifs, les agents bactéricides.

Bien entendu l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

Lorsque la composition est destinée à être appliquée sur les ongles, elle peut se présenter sous forme de vernis à ongles ou de composition de soin des ongles.

Lorsque la composition est destinée à être appliquée sur les cils, elle peut se présenter sous forme de mascara.

La composition selon l'invention peut également être utilisée comme composition capillaire, qui peut être un shampooing, un après-shampooing, une composition de mise en plis, une laque, une lotion coiffante.

La composition selon l'invention peut être conditionnée seule dans un flacon. Elle peut également être conditionnée dans deux récipients séparés, chaque récipient contenant un des premier et second polymères.

On va maintenant donner des exemples illustrant la présente invention sans toutefois la limiter.

### Exemple 1 :

On a préparé un vernis à ongles ayant la composition suivante :

| | |
|---|---|
| - Résine fluorée en solution à 60% en poids dans le xylène vendue sous la dénomination "LUMIFLON LF 200" par la société ICI - (Premier polymère; γ₁ = 35,7 mJ/m²) | |
| | 33,3 g |
| - Polyuréthanne en solution à 25 % en poids dans un mélange toluène/isopropanol/acétone vendu sous la dénomination "DESMOLAC 4125" par la société BASF (Second polymère; γ₂ = 31 mJ/m²) | |
| | 33,3 g |
| - Additifs | 0,5 g |
| - Solvant | qsp 100 g |

Après application de la composition sur l'ongle, on obtient un film brillant, souple et résistant qui se présente sous la forme de deux couches de polymères.

### Exemple 2 :

On a préparé un vernis à ongles ayant la composition suivante :

| | |
|---|---|
| - Résine fluorée en solution à 60% en poids dans le xylène vendue sous la dénomination "LUMIFLON LF 200" par la société ICI - (Premier polymère; γ₁ = 35,7 mJ/m²) | |
| | 25 g |
| - Polymère acrylique en solution à 40 % dans le xylène vendu sous la dénomination "NEOCRYL B 700" par la société ICI - (Second polymère; γ₂ = 30,8 mJ/m²) | |
| | 37,5 g |
| - Pigments | 1 g |
| - Additif rhéologique | 1 g |
| - Additifs | 0,5 g |
| - Solvant | qsp 100 g |

Après application de la composition sur l'ongle, on obtient un film brillant, souple et résistant qui se présente sous la forme de deux couches de polymères.

## Revendications

1. Composition susceptible d'être appliquée sur un support kératinique, caractérisée par le fait qu'elle comprend un premier polymère filmogène ayant une tension superficielle γ₁ proche de la tension superficielle dudit support kératinique sur lequel ladite composition est appliquée, et un second polymère filmogène ayant une tension superficielle γ₂ inférieure à γ₁, les deux polymères filmogènes formant au moins deux phases différentes dans le film sec obtenu après l'application de ladite composition.

2. Composition selon la revendication 1, caractérisée par le fait que γ₁ est proche de la tension superficielle de l'ongle.

3. Composition selon la revendication 2, caractérisée par le fait que γ₁ va de 30 à 35 mJ/m² et/ou γ₂ va de 22 à 32 mJ/m² et γ₂ est inférieur à γ₁.

4. Composition selon la revendication 1, caractérisée par le fait que γ₁ est proche de la tension superficielle du cheveu, du cil ou du sourcil.

5. Composition selon la revendication 4, caractérisée par le fait que γ₁ va de 28 à 33 mJ/m² et/ou γ₂ va de 22 à 32 mJ/m² et γ₂ est inférieur à γ₁.

6. Composition selon l'une quelconque des revendications précédentes, caractérisée par le fait que la teneur en matière sèche de polymères filmogènes va de 5 % à 50 % en poids par rapport au poids total de la composition.

7. Composition selon l'une quelconque des revendications précédentes, caractérisée par le fait qu'elle comprend un mélange de polymères filmogènes constitué, en matière sèche, de 5 % à 95 % en poids d'un premier polymère filmogène et de 5 % à 95 % d'un second polymère filmogène, par rapport au poids total dudit mélange.

8. Composition selon l'une quelconque des revendications précédentes, caractérisée par le fait que les polymères filmogènes sont choisis parmi les polymères radicalaires, les polycondensats, les polymères d'origine naturelle et leurs mélanges.

9. Composition selon la revendication 8, caractérisée par le fait que les polymères radicalaires sont choisis parmi les polymères acryliques et/ou les polymères vinyliques.

10. Composition selon la revendication 8, caractérisée par le fait que les polycondensats sont choisis parmi les polyuréthannes, les polyesters, les polyesters amides, les polyamides, les polyesters à chaînes grasses, les résines époxyesters et leurs mélanges.

11. Composition selon la revendication 8, caractérisée par le fait que les polymères d'origine naturelle sont choisis parmi la shellac et/ou la gomme de sandaraque.

12. Composition selon l'une quelconque des revendications précédentes, caractérisée par le fait qu'elle contient au moins un solvant choisi parmi les solvants organiques, l'eau et leurs mélanges.

13. Composition selon l'une quelconque des revendications précédentes, caractérisée par le fait qu'elle se présente sous forme de vernis à ongles, de composition de soin des ongles.

14. Composition selon l'une quelconque des revendications 1 à 12, caractérisée par le fait qu'elle se présente sous forme de mascara.

15. Composition selon l'une quelconque des revendications 1 à 12, caractérisée par le fait qu'elle se présente sous forme de composition capillaire.

16. Utilisation dans une composition à appliquer sur un support kératinique d'un premier polymère filmogène ayant une tension superficielle γ₁ proche de la tension superficielle dudit support kératinique sur lequel ladite composition est appliquée, et un second polymère filmogène ayant une tension superficielle γ₂ inférieure à γ₁, les deux polymères filmogènes formant au moins deux phases différentes dans le film sec obtenu après l'application de ladite composition.

17. Utilisation selon la revendication 16 afin d'obtenir sur le support un film stratifié.

18. Utilisation selon la revendication 16 dans une composition de vernis à ongles, de soin des ongles, de mascara, ou dans une composition capillaire.

## Claims

1. Composition capable of being applied on a keratinous substrate, characterized in that it comprises a first film-forming polymer having a surface tension γ₁ similar to the surface tension of the said keratinous substrate on which the said composition is applied and a second film-forming polymer having a surface tension γ₂ which is less than γ₁, the two film-forming polymers forming at least two different phases in the dry film obtained after application of the said composition.

2. Composition according to Claim 1, characterized in that γ₁ is similar to the surface tension of the nail.

3. Composition according to Claim 2, characterized in that γ₁ ranges from 30 to 35 mJ/m² and/or γ₂ ranges from 22 to 32 mJ/m² and γ₂ is less than γ₁.

4. Composition according to Claim 1, characterized in that γ₁ is similar to the surface tension of the hair, the eyelash or the eyebrow.

5. Composition according to Claim 4, characterized in that γ₁ ranges from 28 to 33 mJ/m² and/or γ₂ ranges from 22 to 32 mJ/m² and γ₂ is less than γ₁.

6. Composition according to any one of the preceding claims, characterized in that the content on a dry basis of film-forming polymers ranges from 5% to 50% by weight with respect to the total weight of the composition.

7. Composition according to any one of the preceding claims, characterized in that it comprises a mixture of film-forming polymers composed, on a dry basis, of 5% to 95% by weight of a first film-forming polymer and of 5% to 95% of a second film-forming polymer, with respect to the total weight of the said mixture.

8. Composition according to any one of the preceding claims, characterized in that the film-forming polymers are chosen from radical polymers, polycondensates, polymers of natural origin and their mixtures.

9. Composition according to Claim 8, characterized in that the radical polymers are chosen from acrylic polymers and/or vinyl polymers.

10. Composition according to Claim 8, characterized in that the polycondensates are chosen from polyurethanes, polyesters, polyesteramides, polyamides, polyesters containing fatty chains, epoxy- ester resins and their mixtures.

11. Composition according to Claim 8, characterized in that the polymers of natural origin are chosen from shellac and/or sandarac gum

12. Composition according to any one of the preceding claims, characterized in that it contains at least one solvent chosen from organic solvents, water and their mixtures.

13. Composition according to any one of the preceding claims, characterized in that it is provided in the form of a nail varnish or of a nail care composition.

14. Composition according to any one of Claims 1 to 12, characterized in that it is provided in the form of a mascara.

15. Composition according to any one of Claims 1 to 12, characterized in that it is provided in the form of a hair composition.

16. Use, in a composition to be applied on a keratinous substrate, of a first film-forming polymer having a surface tension γ₁ similar to the surface tension of the said keratinous substrate on which the said composition is applied and of a second film-forming polymer having a surface tension γ₂ which is less than γ₁, the two film-forming polymers forming at least two different phases in the dry film obtained after application of the said composition.

17. Use according to Claim 16, in order to obtain a laminated film on the substrate.

18. Use according to Claim 16 in a nail varnish composition, a nail care composition or a mascara composition or in a hair composition.

## Patentansprüche

1. Zusammensetzung, die auf einen Keratinträger aufgebracht werden kann, dadurch gekennzeichnet, daß sie ein erstes filmbildendes Polymer mit einer Oberflächenspannung γ₁ in der Nähe der Oberflächenspannung des Trägers, auf den das Polymer aufgebracht wird, und ein zweites filmbildendes Polymer mit einer Oberflächenspannung γ₂ unter γ₁ enthält, wobei die beiden filmbildenden Polymere in dem nach dem Auftragen der Zusammensetzung erhaltenen trockenen Film mindestens zwei verschiedene Phasen bilden.

2. Zusammensetzung nach Anspruch 1, dadurch gekennzeichnet, daß γ₁ in der Nähe der Oberflächenspannung des Nagels liegt.

3. Zusammensetzung nach Anspruch 2, dadurch gekennzeichnet, daß γ₁ im Bereich von 30 bis 35 mJ/m² und/oder γ₂ im Bereich von 22 bis 32 mJ/m² und γ₂ unter γ₁ liegt.

4. Zusammensetzung nach Anspruch 1, dadurch gekennzeichnet, daß γ₁ in der Nähe der Oberflächenspannung der Haare, der Wimpern oder der Augenbrauen liegt.

5. Zusammensetzung nach Anspruch 4, dadurch gekennzeichnet, daß γ₁ im Bereich von 28 bis 33 mJ/m² und/oder γ₂ im Bereich von 22 bis 32 mJ/m² und γ₂ unter γ₁ liegt.

6. Zusammensetzung nach einem der vorhergehenden Ansprüche, dadurch gekennzeichnet, daß der Trockensubstanzgehalt der filmbildenden Polymere im Bereich von 5 bis 50 Gew.-%, bezogen auf das Gesamtgewicht der Zusammensetzung, liegt.

7. Zusammensetzung nach einem der vorhergehenden Ansprüche, dadurch gekennzeichnet, daß sie ein Gemisch von filmbildenden Polymeren enthält, das aus 5 bis 95 Gew.-% Trockensubstanz eines ersten filmbildenden Polymers und aus 5 bis 95 Gew.-% Trockensubstanz eines zweiten filmbildenden Polymers, bezogen auf das Gesamtgewicht des Gemisches, besteht.

8. Zusammensetzung nach einem der vorhergehenden Ansprüche, dadurch gekennzeichnet, daß die filmbildenden Polymere unter den radikalischen Polymeren, Polykondensaten, Polymeren natürlichen Ursprungs und deren Gemischen ausgewählt sind.

9. Zusammensetzung nach Anspruch 8, dadurch gekennzeichnet, daß die radikalischen Polymere unter den Acrylpolymeren und/oder den Vinylpolymeren ausgewählt sind.

10. Zusammensetzung nach Anspruch 8, dadurch gekennzeichnet, daß die Polykondensate unter den Polyurethanen, Polyestern, Polyesteramiden, Polyamiden, Polyestern mit Fettkette, Epoxyesterharzen und deren Gemischen ausgewählt sind.

11. Zusammensetzung nach Anspruch 8, dadurch gekennzeichnet, daß die Polymere natürlichen Ursprungs unter Schellack und/oder Sandarak ausgewählt sind.

12. Zusammensetzung nach einem der vorhergehenden Ansprüche, dadurch gekennzeichnet, daß sie mindestens ein Lösungsmittel enthält, das unter den organischen Lösungsmitteln, Wasser und deren Gemischen ausgewählt ist.

13. Zusammensetzung nach einem der vorhergehenden Ansprüche, dadurch gekennzeichnet, daß sie in Form von Nagellack oder einer Zusammensetzung zur Pflege der Nägel vorliegt.

14. Zusammensetzung nach einem der Ansprüche 1 bis 12, dadurch gekennzeichnet, daß sie in Form von Mascara vorliegt.

15. Zusammensetzung nach einem der Ansprüche 1 bis 12, dadurch gekennzeichnet, daß sie in Form einer Zusammensetzung für das Haar vorliegt.

16. Verwendung eines ersten filmbildenden Polymers mit einer Oberflächenspannung γ₁ in der Nähe der Oberflächenspannung des Keratinträgers, auf den das Polymer aufgebracht wird, und eines zweiten filmbildenden Polymers mit einer Oberflächenspannung γ₂ unter γ₁ in einer Zusammensetzung, die auf einen Keratinträger aufgebracht werden soll, wobei die beiden filmbildenden Polymere in dem nach dem Auftragen der Zusammensetzung erhaltenen trockenen Film mindestens zwei verschiedene Phasen bilden.

17. Verwendung nach Anspruch 16, um auf dem Träger einen geschichteten Film herzustellen.

18. Verwendung nach Anspruch 16 in einer Nagellack-Zusammensetzung, einer Zusammensetzung zur Pflege der Nägel, Mascara oder einer Zusammensetzung für das Haar.
